# EUROPEAN PATENT APPLICATION

(11) **EP 4 516 311 A1**
(43) Date of publication of application: **05.03.2025**
(21) Application number: 23730697.2
(22) Date of filing: 21.04.2023
(51) Int. Cl.: A61K 39/00

(54) **NUCLEIC ACID SEQUENCE ENCODING A CHIMERIC ANTIGEN NATURAL KILLER CELL RECEPTOR (NK-CAR), POLYPEPTIDE OF SAID NK-CAR, VECTOR COMPRISING SAID NUCLEIC ACID SEQUENCE, IN VITRO METHOD OF OBTAINING AN NK CELL, USE OF SAID NUCLEIC ACID SEQUENCE, POLYPEPTIDE OR VECTOR, AND PHARMACEUTICAL COMPOSITION**

(30) Priority: 29.04.2022 BR 102022008333
(71) Applicant: Fundação Hemocentro de Ribeirão Preto, 14051-140 Ribeirão Preto, SP (BR); Universidade de São Paulo, São Paulo/SP, CEP: 05508-220 (BR)
(72) Inventor: COVAS, Dimas Tadeu, 14051-140 Ribeirao Preto - SP (BR); CASTRO, Virginia Picanço E, 14051-140 Ribeirao Preto - SP (BR); RODRIGUES, Rodrigo Do Tocantins Calado De Saloma, 14051-140 Ribeirao Preto - SP (BR); SILVESTRE, Renata Nacasaki, 14051-140 Ribeirao Preto - SP (BR); AZEVEDO, Julia Teixeira Cottas De, 14051-140 Ribeirao Preto (BR)
(74) Representative: Schlich
(86) International application number: PCT/BR2023/050127
(87) International publication number: WO 2023/205868

(57) **Abstract**

The present invention refers to a nucleic acid sequence encoding a chimeric antigen natural killer cell receptor (NK-CAR), wherein the NK-CAR comprises: (a) an anti-CD19 single-chain variable fragment (scFv); (b) a transmembrane domain; (c) a 4-1BB co-stimulatory domain; and (d) an intracellular T-cell signaling domain of CD3ζ, wherein the said nucleic acid sequence further comprises an autocleavage peptide and a transgene encoding at least one selected from the group consisting of IL-15RA and IL-27 for use in cancer treatment.

## Description

### Field of the application:

The present invention falls within the field of medical sciences and genetic engineering, more specifically, in the area of antineoplastic agents, since it refers to a chimeric antigen natural killer cell receptor construct (NK-CAR) which modulates the interleukin-15 (IL-15) and interleukin-27 (IL-27) pathways for use in cancer treatment.

### Background of the invention and State of the art:

Cell therapy is an innovative approach that allows numerous possibilities in the field of cancer treatment. T cells genetically modified with a chimeric antigen receptor (CAR) have been successfully used in relapsed / refractory hematologic patients.

Without a doubt, T-CAR cell therapy has proven its importance in fighting cancer, with one caveat: this therapy requires sufficient functional primary T-cells for reinfusion into the patient. The expansion of T cells to sufficient T-CAR cells for autologous treatment is a time-consuming process and the efficiency of transduction and expansion is uncertain. These parameters will depend a lot on the quality of these cells coming from patients, who are often severely affected and do not have many healthy T-cells. In addition, T-CAR therapy still has a long way to go to be effective against solid tumors. Thus, all these challenges highlight the need to look for other immunotherapy options, and recent developments have shown that Natural Killers (NK) cell therapy is one of the most promising.

NK-CAR cells have several advantages over T-CAR cells. First, unlike T-CAR cells, NK-CAR cells retain an intrinsic ability to recognize and target their cytotoxicity to tumor cells through their native receptors, making tumor cell evasion less likely through negative regulation of CAR target antigen. Second, NK-CAR cells do not undergo clonal expansion *in vivo* or immune rejection. Unlike T-CAR cells, NK cells do not cause cytokine release syndrome (CRS) because they produce a different cytokine profile than T-cells. NK cells usually produce cytokines such as gamma interferon (IFN-γ) and granulocyte and macrophage colony stimulating factor (GM-CSF). T cells, on the other hand, produce a large amount of inflammatory cytokines, such as tumor necrosis factor (TNF) α**,** interleukin (IL) 6 and IL-1, causing CRS.

Finally, an important risk of T-cell-CAR immunotherapy, for the case of a possible allogeneic treatment, is graft-versus-host disease (GVHD). GVHD has as its main mechanism the alloreactivity of T cells to host cells, but it occurs by different mechanisms depending on the type of the T cell. In general, donor CD8+ T cells are activated when their T-cell receptor (TCR) binds to peptides presented by the host major histocompatibility complex (MHC) class I. The cytotoxic effects of CD8+ T cells are mediated by secretion of perforin and granzyme, and by FasL. Donor CD4+ T cells, on the other hand, are activated when the TCR binds to peptides presented by host antigen-presenting cells (APC) via MHC class II. The response triggered by activation of CD4+ T cells is the one that can cause a T helper 1 (Th1) or T helper 2 (Th2) inflammatory response.

Due to the fact that they do not express TCR, NK cells generally do not cause GVHD, which makes it possible that NK-CAR cells are ready for allogeneic therapeutic use (*off-the-shelf*)*.*

In addition to causing the direct killing of tumor cells, NK cells produce cytokines and chemokines that help in the activation and recruitment of dendritic cells to the tumor environment, allowing these cells to recognize tumor antigens released by lysed target cells and present them to T cells, amplifying the response against the tumor.

Thus, cytokines play a key role in activating cells of the immune system, including NK cells. The incorporation of cytokines into CAR, originating from the fourth generation CAR, may represent an increase in the potency of NK-CAR cells.

However, with T-CAR cells, NK-CAR cell therapy still must overcome several difficulties, such as antigen target loss, tumor heterogeneity and hostile tumor microenvironment. In addition, survival and expansion of NK cells are still challenging obstacles. Thus, the ability to target NK cell cytotoxicity against refractory tumors by CAR expression will likely contribute to a change in basic assumptions in the cancer treatment.

In this sense, with a view of solving the technical problems cited, the present invention proposes a chimeric antigen natural killer cell receptor construct (NK-CAR) which modulates the interleukin-15 (IL-15) and interleukin-27 (IL-27) pathways for use in cancer treatment. These CAR constructs which modulate the proposed IL-15 or IL-27 pathways can promote significant increases in proliferation, activation, secretion of cytokines and tumor cytolytic activity of NK cells. Besides, the present invention proposes a novel vector containing a fusion of IL-15 with its receptor R alpha, targeting a better persistence of NK-CAR cell activity.

Although there are many clinical trials using T-ARC cells, a few documents of the state of the art describe clinical trials for NK-ARC cells.

The scientific article entitled *"*Soluble interleukin-15 receptor alpha (IL-15R alpha)-sushi as a selective and potent agonist of IL-15 action through IL-15R beta/gamma. Hyperagonist IL-15 x IL-15R alpha fusion proteins", by Mortier et al., published on January 20, 2006 in the magazine J Biol Chem., 281(3): 1612-9, under doi: 10.1074/jbc.M50862420, discloses that the use of a fusion protein comprising IL-15 linked to an IL-15Rαsushi domain via a flexible ligand can provide more potent activity on lymphocyte proliferation (such as NK cells, NK-T cells and CD8-positive memory cells), dendritic cell activation and similar than that caused by the conventional combined use of the IL-15 and IL-15Rαsushi domain. However, such an article by Mortier et al. is silent on specific descriptions of combinations of IL-15 and IL-15Rαsushi with CAR.

The scientific article entitled *"*Expression of IL-15RA or an IL-15/IL-15RA fusion on CD8+ T cells modifies adoptively transferred T-cell function in cis", by Rowley et al., published on February, 2009 in magazine Eur J Immunol., 39(2): 491-506, under doi: 10.1002/eji.200838594, discloses that the transfection of a mouse IL-15 and IL-15Rα secretory fusion protein improves the viability and proliferative capacity of CD8-positive T cells. However, this article by Rowley et al. includes neither NK cells nor specific descriptions of combinations of IL-15 with CAR.

The scientific article entitled *"*Treatment with IL-27 attenuates experimental colitis through the suppression of the development of IL-17-producing T helper cells", by Sasaoka et al., published on December 30, 2010, in magazine Am J Physiol Gastrointest Liver Physiol, 300: G568-G576, under doi:10.1152/ajpgi.00329.2010, discloses a single-chain IL-27 (p28 and EBI3 linked by a flexible linker) and an effect of it in the treatment of inflammatory bowel disease (IBD). However, this article by Sasaoka et al. does not include descriptions of CAR-NK cells and much less combinations of IL-27.

The international patent application no. PCT/US2005/036407, published by the no. WO 2007/037780 on April 5, 2007, in the name of GOVERNMENT OF THE UNITED STATES OF AMERICA, represented by THE SECRETARY, DEPARTMENT OF HEALTH AND HUMAN SERVICES, entitled: "*Adoptive immunotherapy with enhanced T lymphocyte survival"* describes T cells expressing recombinant IL-7, recombinant IL-15 or combinations of these and reveals that expression of such cytokines improves T cell survival. However, such an international application does not include specific descriptions of CAR-NK cells and neither does it include combinations of specific cytokines with CAR.

The international patent application no. PCT/US2012/055443, published as WO 2013/040371 on March 21, 2013, in name of BAYLOR COLLEGE OF MEDICINE, entitled: *"Targeting the tumor microenvironment using manipulated NKT cells"* reveals a modified T cell comprising an expression construct encoding IL-2, IL-4, IL-7, IL-15 or combinations thereof and a CAR construct. However, such international application do not include descriptions of combinations of IL27 cytokines and neither IL15-IL15Ra.

The international patent application no. PCT/US2014/038005, published as WO 2014/186469 on November 20, 2014, in name of BOARD OF REGENTS, THE UNIVERSITY OF TEXAS SYSTEM, entitled: *"Human application of engineered chimeric antigen receptor (CAR) T-cells"* discloses CAR-T cells expressing cytokines linked to the membrane, such as IL-7, IL-15 (fusion protein IL-15/IL15Rα) and IL-21. However, such international application does not include specific descriptions of combinations with IL-27 and neither the use on NK cells.

The US patent application no. US 2013/071414, published on March 21, 2013, in name of DOTTI GIANPIETRO, SPENCER DAVID M, ROONEY CLIONA M, and BRENNER MALCOLM K, entitled: *"Engineered CD19-specific T lymphocytes that co-express IL-15 and an inducible CASPASE-9 based suicide gene for the treatment of B-cell malignancies"* discloses a CAR-T cell expressing IL-15 and targeting CD19. However, such US application does not include specific descriptions of combinations of IL-15-IL15RA, or IL 27 in NK cells.

The European patent application no. EP 3 845 654, published on July 7, 2021, in name of Noile-Immune Biotech, Inc, and Takeda Pharmaceutical Company Limited, entitled: *"CAR-expressing T cells and CAR expression vector"* discloses a CAR-T cell expressing IL-15 or IL-27 on T cells in combination with the CCL19 chemokine. Differently, the present invention proposes an NK-CAR construct wherein no chemokines are used.

The scientific article entitled *"*Glypican-3-specific CAR T cells co-expressing IL15 and IL21 have superior expansion and antitumor activity against hepatocellular carcinoma", by Barta et al., published on January 17, 2020, in magazine Cancer Immunol Res., 8(3): 309-320, under doi: 10.1158/2326-6066.CIR-19-0293, reveals a CAR-T cell which expresses IL-15 and / or IL-21 and directs GPC3. However, this article does not include specific descriptions of combinations with IL-15-IL15RA or with IL-27, thus distancing itself from, of the present invention.

Thus, differently from the prior art, it is an objective of the present invention to provide allogeneic immune cells (such as CAR-NK cells) with enhanced antitumor activity by co-expressing cytokines. Therefore, the antitumor activity by CAR can be increased (for example, reduction in the number of residual tumor cells, improvement in the amount of IFNγ to be produced and improvement in migration and accumulation of host immune cells (such as T cells, dendritic cells, NK cells) at the tumor site). In addition, the therapeutic effect on the cancer can be enhanced by a drug comprising the NK cell of the present invention.

Thus, no document of the state of the art discloses a chimeric antigen natural killer cell receptor construct (NK-CAR) which modulates the interleukin-15 (IL-15) and interleukin-27 (IL-27) pathways for use in cancer treatment, such as proposed by the present invention.

### Summary of the invention:

The present invention will provide significant advantages over cell therapy for the treatment of neoplasms.

In a first aspect, the present invention refers to a nucleic acid sequence encoding a chimeric antigen natural killer cell receptor (NK-CAR), wherein the NK-CAR comprises:
(a) an anti-CD19 single-chain variable fragment (scFv);
(b) a transmembrane domain;
(c) a 4-1BB co-stimulatory domain; and
(d) an intracellular T-cell signaling domain of CD3ζ,
wherein the said nucleic acid sequence further comprises an autocleavage peptide and a transgene encoding at least one selected from the group consisting of interleukin-15 with its receptor RA (IL-15RA) and interleukin-27 (IL-27).

In a second aspect, the present invention additionally refers to a polypeptide of the chimeric antigen natural killer cell receptor (NK-CAR) comprising:
(a) an anti-CD19 single-chain variable fragment (scFv);
(b) a transmembrane domain;
(c) a 4-1BB co-stimulatory domain; and
(d) an intracellular T-cell signaling domain of CD3ζ,
wherein such polypeptide further comprises an autocleavage peptide and at least one cytokine selected from the group consisting of interleukin-15 with its receptor RA (IL-15RA) and interleukin-27 (IL-27).

In a third aspect, the present invention additionally refers to a vector comprising the nucleic acid sequence of the present invention.

In a fourth aspect, the present invention additionally refers to an *in vitro* method of obtaining a cell comprising the following steps: (a) transforming a cell with the vector of the invention; and (b) culturing such transformed cell under conditions of cell growth, wherein such cell is a Natural-Killer (NK) cell.

In a fifth aspect, the present invention additionally refers to the use of the said nucleic acid sequence of the invention, of such NK-CAR polypeptide of the invention, or of such vector of the invention for the preparation of a drug to treat the cancer.

In a sixth aspect, the present invention additionally refers to a pharmaceutical composition comprising the vector of the chimeric antigen natural killer cell receptor (NK-CAR) of the present invention, or the polypeptide of the invention, or the nucleic acid sequence of the invention and a pharmaceutically acceptable vehicle.

### Brief description of the figures:

The present invention, together with its additional advantages, can be better understood by referring to the attached images and the following description.
Figure 1 refers to the lentiviral vectors used for evaluation of CAR.19 expression and effect of cytokines.
Figure 2 represents the electrophoresis of the enzymatic digestion of lentiviral vectors and the respective size of the DNA bands.
Figures 3A-D graphically show CAR expression and stability in NK-92 cells transduced with the SEW-SFFV-CAR.19, SEW-SFFV-CAR.19-IL-15, SEW-SFFV-CAR.19-IL-15/IL-15Rα and SEW-SFFV-CAR.19-IL-27 constructs.
Figures 4A-E graphically represent the percentage of positive cells for NK-92 markers: CD56, CD45, CD28, CD11a (LFA-1), CD2 (LFA-2), NKG2D, NKp30, NKp46, CD95 in cells: (A) NK-92 wt, (B) NK-92-SFFV-CAR.19, (C) NK-92-SFFV-CAR.19-IL-15, (D) NK-92-SFFV-CAR.19-IL-15/IL-15Rα and (E) NK-92-SFFV-CAR.19-IL-27 (n = 2).
Figures 5A-D graphically represent the cell proliferation of NK-92 cell lines modified with different CAR.19 constructs expressing interleukins, in the presence or absence of IL-2.
Figures 6A-C graphically represent the cytotoxic effect of NK-92-CAR cells against CD19+ (NALM-6 and Raji) and CD19- (K562).
Figures 7A-J graphically represent the cytokines secreted in co-culture assay of NK-92 effector cells wt or NK-92 transduced with different CAR.19 constructs and Raji, NALM-6 and K562 target cells.
Figures 8A-C graphically represent the cell exhaustion test (*re-challenging*) of NK-92 wt, NK-92-SFFV-CAR.19, NK-92-SFFV-CAR.19-IL-15, NK-92-SFFV-CAR.19-IL-15/IL-15Rα, NK-92-SFFV-CAR.19-IL-27 cells exposed to the Raji target cell in the ratio of 1:2 (effector cells: target cells) .
Figures 9A-C graphically represent the transduction of primary PB NK cells with SEW-SFFV-CAR.19-IL-15, SEW-SFFV-CAR.19-IL-15/IL-15Rα and SEW-SFFV-CAR.19-IL-27 vectors and cell proliferation.
Figures 10A-B graphically represent the cytotoxicity assay for NK-PB-SFFV-CAR.19-IL-15, NK-PB-SFFV-CAR.19-IL-15/IL-15Rα and NK-PB wt effector cells against CD19 positive tumor cells (triplicate).
Figures 11A, 11C and 11E represent the evaluation of the cytotoxic potential of NK-92-SFFV-CAR.19 (4 constructs) and NK-92 wt cells in NSG mouse models with infusion of 4×10⁴ Raji-luc tumor cells (systemic lymphoma model) .
Figures 12A-E represent the evaluation of the cytotoxic potential of NK-92-SFFV-CAR.19-IL-15/IL-15Rα and NK-92 wt cells in NSG mouse models after IV infusion of 2×10⁴ Raji-luc tumor cells (systemic lymphoma model).

### Detailed description of the invention:

Although the present invention may be susceptible to different embodiments, a preferred embodiment is shown in the following detailed description, with the understanding that the present embodiment should be considered an exemplification of the principles of the invention and is not intended to limit the present invention to what has been described in this specification.

The present invention refers to a nucleic acid sequence encoding a chimeric antigen natural killer cell receptor (NK-CAR), wherein the NK-CAR comprises:
(a) an anti-CD19 single-chain variable fragment (scFv);
(b) a CD8 transmembrane domain;
(c) a 4-1BB co-stimulatory domain; and
(d) an intracellular T-cell signaling domain of CD3ζ,
wherein the said nucleic acid sequence further comprises a T2A autocleavage peptide and a transgene encoding at least one selected from the group consisting of interleukin-15 with its receptor RA (IL-15RA) and interleukin-27 (IL-27).

In one embodiment of the invention, the scFV anti-CD19 comprises the amino acid sequence as set out in the SEQ ID NO: 1, wherein it is encoded by the nucleotide sequence as set out in SEQ ID NO: 2.

Additionally, the amino acids in positions 1 to 20 of SEQ ID NO: 1 refer to the IL-2 signal peptide and the nucleotides in positions 1 to 60 of SEQ ID NO: 2 refer to a nucleotide sequence encoding the IL-2 signal peptide.

The signal peptide is an amino acid sequence usually located in the N-terminal region of proteins. Many of the proteins synthesized in cellular compartments do not necessarily play their biological roles in the places where they are generated, needing to be exported to the specific region where they will exert their functions. The signal peptide sequence has the function of marking the proteins that will be exported to certain locations, such as the extracellular environment. These proteins are recognized by the signal peptide, which, after export, is removed from the protein by the action of proteases. Signal peptides can also be composed of sequences located internally in proteins, which are not subsequently removed, but remain as an integral part of the protein.

In one embodiment of the invention, the transmembrane domain comprises the amino acid sequence as set out in the SEQ ID NO: 3, wherein it is encoded by the nucleotide sequence as set out in SEQ ID NO: 4.

In one embodiment of the invention, the co-stimulatory domain comprises the amino acid sequence as set out in the SEQ ID NO: 5, wherein it is encoded by the nucleotide sequence as set out in SEQ ID NO: 6.

In one embodiment of the invention, the intracellular T-cell signaling domain of CD3ζ comprises the amino acid sequence as set out in the SEQ ID NO: 7, wherein it is encoded by the nucleotide sequence as set out in SEQ ID NO: 8.

Such autocleavage peptide is selected from the group consisting of P2A, E2A, F2A and T2A.

In one embodiment of the invention, the heterologous T2A autocleavage peptide comprises the amino acid sequence as set out in SEQ ID NO: 9, wherein it is encoded by the nucleotide sequence as set out in SEQ ID NO: 10.

In one embodiment of the invention, the transgene encoding at least one selected from the group consisting of interleukin-15 with its receptor RA (IL-15RA) and interleukin-27 (IL-27) comprises the nucleotide sequence as set out in SEQ ID Nos: 11 and 12, respectively. Also, the IL-15RA and IL-27 encoded by such transgene comprises the amino acid sequences as set out in SEQ ID Nos: 13 and 14, respectively.

In view of the above, the proposed NK-CAR is considered a fourth generation CAR designed to secrete a cytokine together with CAR signaling in the target tumor tissue, thus achieving a more potent CAR.

Fourth generation CAR has several advantages: (i) the cytokine of interest is deposited in the tumor region (target of the CAR), (ii) inducible cytokine release avoids systemic toxicity while achieving the therapeutic dose in the target tissue, (iii) in the case of continuous cytokine release, the cytokine can reach high levels in the long term, provided that the fourth generation producing CAR cell is activated, (iv) it can promote the initiation of a secondary immune response against cancer cells that is invisible to NK-CAR cells and at last (v) low numbers of fourth generation CAR cells are required to produce the same antitumor effect as NK-CAR cells without transgenic cytokine.

Cytokines are crucial natural adjuvants involved in the regulation and activation of NK cells against tumor cells. Some of these stimulatory factors are IL-12, IL-15, IL-2, IFN-α and IFN-β. Members of the TNF family of cytokines are expressed by NK cells and important mediators of apoptosis.

IL-15 is a pleiotropic cytokine essential for the development and function of NK cells and is currently under investigation as an immunotherapeutic agent for the treatment of cancer. IL-15 is an interleukin highly related to IL-2, with its own role in the development, survival, proliferation and activation of NK cells and lymphocytes. Due to these properties, IL-15 has been used in several pre-clinical and clinical studies, the last involving the use of IL-15 for the treatment of hematological malignancies and solid tumors.

Recently, the creation of the IL-15 super agonist by coupling IL-15 to its soluble high-affinity alpha receptor (IL-15 Rα), inspired by the natural trans-presentation of IL-15, has increased the potential of this interleukin. The IL-15 super agonist (IL-15 complexed with IL-15 receptor alpha (IL-15 Rα)) shows promising advantages over monomeric IL-15 by exhibiting prolonged half-life and more potently stimulating NK cells. Simultaneous expression of IL-15Rα on the same cell has been shown to be physiologically necessary for the production and secretion of IL-15.

Another important cytokine that can act on the expansion and increased cytotoxicity of NK cells is IL-27. IL-27 is a heterodimeric cytokine composed of two subunits, encoded by two genes: the EBI3 (*Epstein-Barr virus-induced gene 3*) (chromosome 19) and the IL-27p28 (chromosome 16). IL-27 is expressed by antigen-presenting cells and interacts with a specific cell surface receptor complex known as the IL-27 receptor (IL-27R). This receptor consists of two proteins, IL-27Rα (or WSX1) and gp130. IL-27 induces the differentiation of the various T-cell populations in the immune system.

IL-27 is a cytokine with pro- and anti-inflammatory properties, which promotes human NK cell activation and NKp-46 dependent cytotoxicity. Additionally, IL-27 also conditions NK cells for proper response to IL-18 stimulation and induces increased secretion of IFN-γ. IL-27 provides a feedback mechanism, triggering the secretion of the anti-inflammatory cytokine IL-10 by T cells. However, the role of IL-27 on NK cells is not yet well defined.

It is worth noting that IL-15RA and IL-27 are cytokines that are not expressed by natural NK cells (into which no exogenous genes are introduced). In nature, the DNA of these proteins is not fused, and the proteins interact after translation. In both cytokines (IL15 and IL27) a linker has been added to make this junction, and this does not exist in nature.

In one embodiment, an elastin linker was used to link the EBI3 subunits (amino acids 1 to 687 of the SEQ ID NO: 12) and the IL-27p28 (amino acids 718 to 1362 of the SEQ ID NO: 12) of the cytokine IL-27. Preferably, the linker comprises the nucleotide sequence as set out in SEQ ID NO: 19.

In one embodiment, for the IL-15Rα it was used a linker for joining the IL-15 (1 to 406 amino acids of the SEQ ID NO: 11) with its receptor IL-15Rα (478 to 720 of the SEQ ID NO: 11). Preferably, the linker comprises the nucleotide sequence as set out in SEQ ID NO: 20.

The term "nucleic acid sequence" is intended to encompass a polymer of DNA or RNA, i.e., a polynucleotide, which may be single-chain or double-chain, and which may contain unnatural or altered nucleotides. The term "nucleic acid" as used herein refers to a polymeric form of nucleotides of any length, either ribonucleotides (RNA), or deoxyribonucleotides (DNA). These terms refer to the primary structure of the molecule, and thus include double-chain and single-chain DNA, and double-chain and single-chain RNA. The term includes, as equivalents, analogues of, or RNA, or DNA, produced from nucleotide analogues, and modified polynucleotides, such as, but not limited to, methylated and / or limited polynucleotides.

In one embodiment of the invention, the nucleic acid sequence encoding a NK-CAR comprises the nucleotide sequences of NK-CAR selected from the group consisting of SEQ ID NO: 15 referring to CAR Cd19/IL15RA and SEQ ID NO: 16 referring to CAR Cd19/IL27.

It is worth noting that the nucleotides in positions 1 to 60 of SEQ ID NO: 15 refer to the nucleotide sequence encoding the signal peptide IL-2. The nucleotides in positions 1 to 60 of the SEQ ID NO: 16 refer to the nucleotide sequence encoding the signal peptide IL-2.

Additionally, the present invention refers to a polypeptide of the chimeric antigen natural killer cell receptor (NK-CAR) comprising:
(a) an anti-CD19 single-chain variable fragment (scFv);
(b) a CD8 transmembrane domain;
(c) a 4-1BB co-stimulatory domain; and
(d) an intracellular T-cell signaling domain of CD3ζ,
wherein such polypeptide further comprises a T2A autocleavage peptide and at least one cytokine selected from the group consisting of interleukin-15 with its receptor RA (IL-15RA) and interleukin-27 (IL-27).

In one embodiment of the invention, such NK-CAR polypeptide comprises the amino acid sequences selected from the group consisting of SEQ ID NO: 17 referring to CAR Cd19/IL15RA and SEQ ID NO: 18 referring to CAR Cd19/IL27.

Additionally, the present invention refers to a vector comprising the nucleic acid sequence of the present invention.

In one embodiment of the invention, such vector comprises the nucleic acid sequence as set out in SEQ ID NO: 15 or SEQ ID NO: 16.

Such vector is a lentiviral vector.

Additionally, the present invention refers to an *in vitro* method of obtaining a cell comprising the following steps:
a) transforming a cell with the vector of the invention; and
b) culturing such transformed cells under conditions of cell growth.

Such cell is a Natural-Killer (NK) cell.

Additionally, the present invention refers to the use of an immune effector cell genetically engineered to express a chimeric antigen receptor (CAR) for the preparation of a drug for the destruction of cancer cells, such as the treatment of B-cell cancers, such as lymphoma and leukemia.

Such cell is a Natural-Killer (NK) cell.

In one embodiment of the invention, such use is for allogenic therapeutic use.

Additionally, the present invention refers to a pharmaceutical composition comprising the vector of the chimeric antigen natural killer cell receptor (NK-CAR) of the present invention, or the polypeptide of the present invention, or the nucleic acid sequence of the invention and a pharmaceutically acceptable vehicle.

In one preferred embodiment, the pharmaceutical composition comprises a NK cell expressing the CAR of the invention, more preferably, a population of NK cells expresses the CAR of the invention.

Therefore, in order to elucidate the present invention, the following are examples which additionally illustrate the invention, but naturally should not be construed as in any way limiting its scope.

### Examples:

### - Construction of lentiviral vectors:

The CAR anti-CD19 gene sequences followed by 4-1BB-CD3ζ were synthesized into gene blocks and inserted into the SEW-SFFV vector by molecular cloning using the restriction sites of the enzymes NdeI and SbfI, resulting in the SEW-SFFV-CAR.19 vector. The SFFV promoter of the SEW-SFFV-CAR.19 vector was removed and replaced by the gene sequence from the EF1α promoter by molecular cloning flanked by the restriction enzymes EcoRI and NdeI, resulting in the SEW-EF1α -CAR.19 vector.

Then, three vectors were synthesized containing, in addition to CAR.19 gene sequence, or the sequence of IL-15, or IL-15 together with IL-15Rα or IL-27, using the SEW-SFFV backbone.

The CAR anti-CD19 gene sequences followed by 4-1BB-CD3ζ, T2A and IL-15 were synthesized into gene blocks and inserted into the SEW-SFFV vector by molecular cloning using the restriction sites of the enzymes NdeI and SbfI, generating the vector SEW-SFFV-CAR.19-IL-15. The gene sequences of anti-CD19 CAR followed by 4-1BB-CD3ζ, T2A, IL-15 and IL-15 receptor Rα sequence were synthesized into gene blocks and inserted into the SEW-SFFV vector by molecular cloning using the restriction sites of NdeI and SbfI enzymes, generating the vector SEW-SFFV-CAR.19-IL-15/IL-15Rα. The gene sequences of CAR anti-CD19 followed by 4-1BB-CD3ζ, T2A and IL-27 were synthesized into gene blocks and inserted into the SEW-SFFV vector by molecular cloning using the restriction sites of NdeI and SbfI enzymes, generating the vector SEW-SFFV-CAR.19-IL-27.

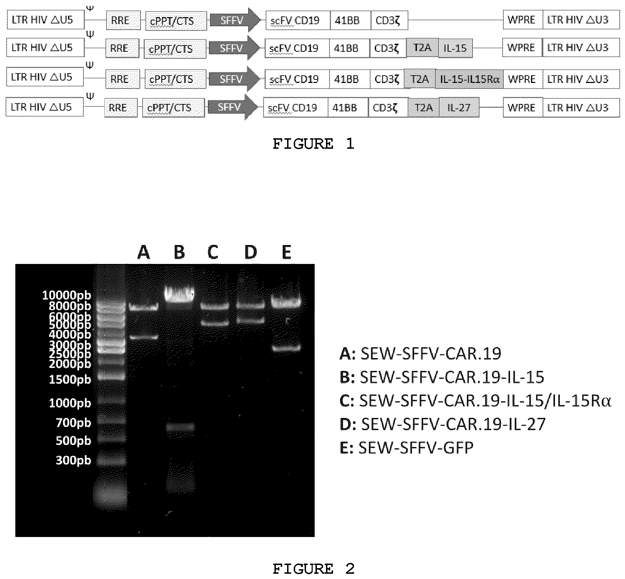

Figure 1 illustrates the aforementioned lentiviral vectors constructed for the evaluation of CAR.19 expression and effect of cytokines, wherein LTR HIV ΔU5 refers to the long terminal 5' self-activated repeat (LTR) of HIV-1; RRE refers to the Ver responsive element; Ψ refers to the packaging signal; cPPT/CTS refers to the central polypurinic tract element; SFFV refers to the promoter of spleen focus-forming virus; scFV refers to the single-chain variable fragment; IL-15 refers to the interleukin 15; IL-27 refers to the interleukin 27; IL-15-IL15Rα refers to the interleukin 15 fused with IL-15 receptor α; WPRE refers to the marmot post-transcriptional regulatory element; and LTR HIV ΔU3 refers to the HIV-1 long terminal 3' self-activated repeat (LTR).

In the lentiviral backbone SEW-SFFV-CAR.19 the cytokines IL-15, IL-15 / IL15-RA (expressing interleukin 15 with the receptor Rα) and IL-27 and the vectors were generated: SEW-SFFV-CAR.19-IL-15, SEW-SFFV-CAR.19-IL-15/IL-15Rα, SEW-SFFV-CAR.19-IL-27. The digestion products of these vectors were separated by electrophoresis in 1 % agarose gel (Figure 2), resulting in sizes of bands close to the expected ones, demonstrating the integrity of the DNA sequence (Table 1).

**Table 1 - Restriction enzymes used for digestion of lentiviral vectors and the expected band sizes.**

| **Lentiviral vectors** | **Enzymes** | **Size of the bands** | **Size of vector (total)** |
|---|---|---|---|
| SEW-SFFV-CAR.19 | Not I | 3171 pb and 7338 pb | 10.509 pb |
| SEW-SFFV-CAR.19-IL-15 | EcoRI | 594 pb and 10320 pb | 10.914 pb |
| SEW-SFFV-CAR.19-IL-15/IL-15Rα | Not I | 4365 pb and 7338 pb | 11.703 pb |
| SEW-SFFV-CAR.19-IL-27 | Not I | 4531 pb and 7338 pb | 11.869 pb |
| SEW-SFFV-GFP | Not I | 2321 pb and 7338 pb | 9.659 pb |

For better understanding, in Figure 2, well A refers to 1 % agarose gel electrophoresis containing DNA bands after enzymatic digestion of the lentiviral vector SEW-SFFV-CAR.19, well B refers to electrophoresis after enzymatic digestion of the lentiviral vector SEW-SFFV-CAR.19-IL-15, well C refers to electrophoresis after enzymatic digestion of the lentiviral vector SEW-SFFV-CAR.19-IL-15Rα; well D refers to electrophoresis after enzymatic digestion of the lentiviral vector SEW-SFFV-CAR.19-IL-27; and well D refers to electrophoresis after enzymatic digestion of the lentiviral vector SEW-SFFV-CAR.19-IL-27. IL-15/IL-15Rα; well D refers to electrophoresis after enzymatic digestion of the lentiviral vector SEW-SFFV-CAR.19-IL-27; and well E refers to electrophoresis after enzymatic digestion of the lentiviral vector SEW-SFFV-GFP; in the first well of the agarose gel a 1 kb molecular weight marker (Axygen).

Thus, the SEW-SFFV-GFP vector was used in the NK-92 cell transduction standardization experiments.

### Experimental tests:

### - Transduction of NK-92 cells with different CAR.19 vectors and positive selection of the cells

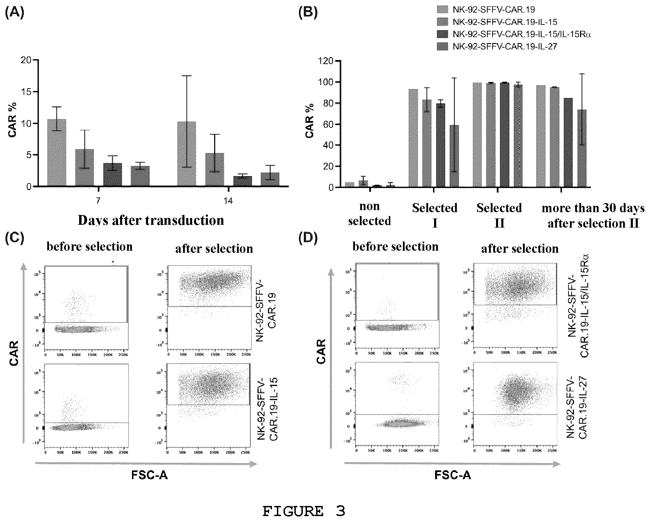

Figures 3A-D graphically show CAR expression and stability in NK-92 cells transduced with the SEW-SFFV-CAR.19, SEW-SFFV-CAR.19-IL-15, SEW-SFFV-CAR.19-IL-15/IL-15Rα and SEW-SFFV-CAR.19-IL-27 constructs, wherein (A) refers to the percentage of NK-92 CAR+ cells transduced with the SEW-SFFV-CAR.19 (n = 2), SEW-SFFV-CAR.19-IL-15 (n = 3), SEW-SFFV-CAR.19-IL-15/IL-15Rα (n = 2) and SEW-SFFV-CAR.19-IL-15Rα (n = 2) and SEW-SFFV-CAR.19-IL-27 (n = 3) constructs on days 07 and 14 after transduction; (B) refers to the enrichment of CAR+ populations after positive selection of NK-92-CAR cells performed in two steps and IL-27 (n = 3) on days 07 and 14 after transduction; (B) refers to the enrichment of CAR+ populations after positive selection of NK-92-CAR cells performed in two steps and evaluation of CAR expression stability after more than 30 days in culture; (C) refers to all dot plots representing flow cytometry data from NK-92-SFFV-CAR.19 and NK-92-SFFV-CAR.19-IL-15 cells before and more than 30 days after the positive selection process of CAR.19 cells; and (D) refer to the dot plots representing flow cytometry data of NK-92-SFFV-CAR.19-IL-15/IL-15Rα and NK-92-SFFV-CAR.19-IL-27 cells before and more than 30 days after the positive selection process of CAR.19 cells.

Therefore, NK-92 cells were transduced with the different vectors and CAR.19 labeling was performed on days 07 and 14. The results showed a transduction efficiency (day 7) of 10.7% ± 1.93 for SEW-SFFV-CAR.19, 5.8 % ± 3 for SEW-SFFV-CAR.19-IL-15, 3.7 % ± 1.1 for SEW-SFFV-CAR.19-IL-15Rα and 3.2 % ± 0.6 for SEW-SFFV-CAR.19-IL-27. It can be seen that the smaller size vectors (SEW-SFFV-CAR.19 and SEW-SFFV-CAR.19-IL-15) (Table 1) show higher transduction efficiency (Figure 3A). Next, in order to increase the percentage of CAR.19 positive NK-92 cells, NK-92 cells expressing CAR on their surface were selected with biotin-conjugated anti-CAR antibody-SP and by anti-biotin microspheres twice. After the second CAR selection, the cells showed CAR expression of about 99% (Figure 3B).

All present constructs showed a fairly stable CAR expression (Figures 3A-D), as measured by surface expression of the percentage of chimeric (CD19-specific) scFv after 30 days in culture (post-selection). CAR expression remained nearly constant for NK-92-SFFV-CAR.19 (97 %), NK-92-SFFV-CAR.19-IL-15 (95 %), NK-92-SFFV-CAR.19-IL-15/IL-15Rα (85 %) and NK-92-SFFV-CAR.19-IL-27 (74 %). These results indicate that CAR expression, driven by the SFFV promoter, remains stable during NK-92 cell expansion. During this period, IL-2 (final concentration of 500 IU/mL) was added to the culture every 2 - 3 days.

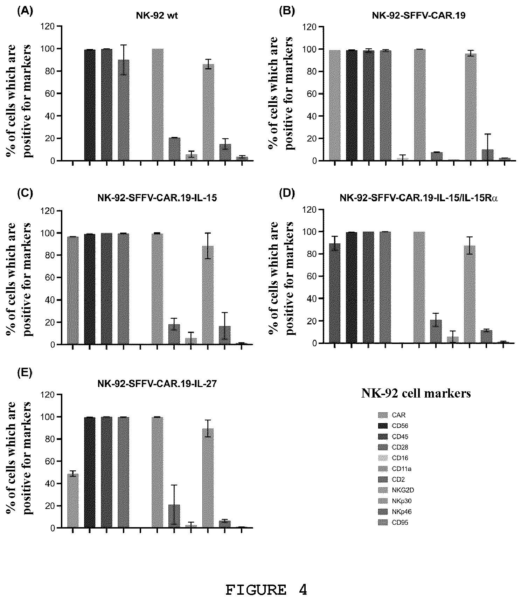

After the generation of NK-92-CAR cells, the immunophenotypic characterization of the different NK-92-CAR cells after expansion was performed (Figures 4A-E).

Accordingly, Figures 4A-E graphically represent the percentage of cells positive for NK-92 markers: CD56, CD45, CD28, CD11a (LFA-1), CD2 (LFA-2), NKG2D, NKp30, NKp46, CD95 in the cells: (A) NK-92 wt, (B) NK-92-SFFV-CAR.19, (C) NK-92-SFFV-CAR.19-IL-15, (D) NK-92-SFFV-CAR.19-IL-15/IL-15Rα and (E) NK-92-SFFV-CAR.19-IL-27 (n = 2); wherein for the genetically modified cells, CAR.19 expression was also evaluated.

In this regard, NK-92 cells wt and all NK-92 cells express CAR.19 and the corresponding interleukins expressed the markers CD56, CD45, CD28, CD11a (LFA-1), NKp30 and less than 50 % of the same cells expressed CD2, CD95, NKG2D and NKp46. The cells did not express CD16, DNAM-1, CD3, CD69. Thus, the results here showed that there was no difference in expression of immunophenotypic markers after transduction of the cells with CAR. (n = 2).

### - Effect of cytokines in the expansion of NK-92-CAR cells

After the stability of CAR expression of the vectors SEW-SFFV-CAR.19, SEW-SFFV-CAR.19-IL-15, SEW-SFFV-CAR.19-IL-15/IL-15Rα and SEW-SFFV-CAR.19-IL-27 was evaluated, it was necessary to analyze the effect of interleukins present in the vector and their effect on the proliferation of the transduced cells. It was further investigated whether the cytokines produced in the vectors would be sufficient to replace exogenous IL-2 and allow cell expansion.

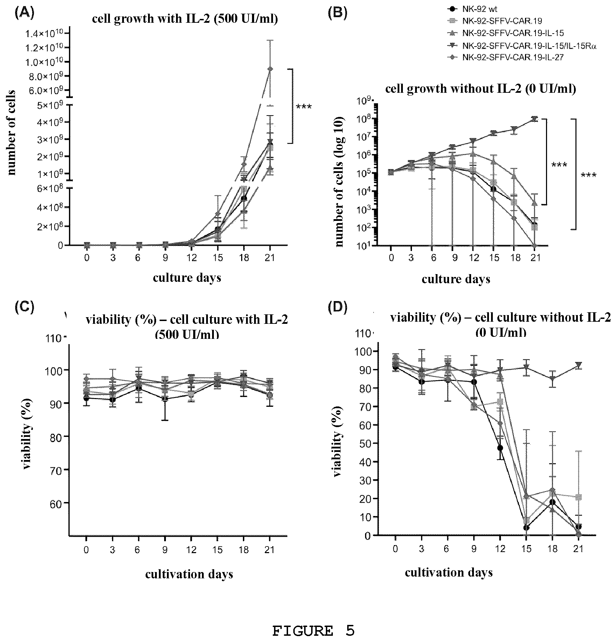

In Figures 5A-D are graphically shown the cell proliferation of NK-92 cell lines modified with different CAR.19 constructs expressing interleukins, in the presence or absence of IL-2. Also shown are NK-92 cells transduced with CAR.19 vectors co-expressing interleukins IL-15 or IL-15 with receptor α or IL-27, wherein (A) refers to the expansion of NK-92 cells wt (n = 4), NK-92-SFFV-CAR.19 (n = 4), NK-92-SFFV-CAR.19-IL-15 (n = 4), NK-92-SFFV-CAR.19-IL-15/IL-15Rα (n = 4) and NK-92-SFFV-CAR.19-IL-27 (n = 4) after 21 days of culture in the presence of IL-2 (500 IU/mL); (B) refers to the expansion of NK-92 wt (n = 4), NK-92-SFFV-CAR.19 (n = 4), NK-92-SFFV-CAR.19-IL-15 (n = 4), NK-92-SFFV-CAR.19-IL-15/IL-15Rα (n = 4) and NK-92-SFFV-CAR.19-IL-27 (n = 4) after 21 days of culture without IL-2; (C) refers to the viability of NK-92 cells cultured in the presence of IL-2 for 21 days; and (D) refers to the viability of NK-92 cells cultured in the absence of IL-2 for 21 days. For these results, the statistical test was considered Two-way ANOVA, Tukey's multiple comparison post-test, and p values < 0,0003 (***).

Therefore, NK-CAR cells with different vectors were cultured in the presence and absence of IL-2 and the proliferation of the cells was evaluated for 21 days, also comparing the proliferation of NK-92 cells wt (Figures 5A and 5B).

In the presence of IL-2 the NK-92-SFFV-CAR.19-IL-27 cells showed a higher cell expansion than the other cells transduced with CAR.19 and the NK-92 wt. Whereas NK-92 wt, NK-92-SFFV-CAR.19, NK-92-SFFV-CAR.19-IL-15, NK-92-SFFV-CAR.19-IL-15/IL-15Rα cells showed very similar cell expansion (Figure 5A).

In the culture condition without IL-2, after 9 days of culture, NK-92 wt, NK-92-SFFV-CAR.19, NK-92-SFFV-CAR.19-IL-27 cells stopped expanding, and the number of cells started to decrease. NK-92-SFFV-CAR.19-IL-15 cells showed positive cell expansion until day 12, and then started to decrease in number. The only cell type that showed growth in number throughout the 21-day period was NK-92-SFFV-CAR.19-IL-15/IL-15Ra, although it had lower growth than the condition with IL-2 (Mean= 2.8 x 10⁹ vs. mean= 9.7 x 10⁷ in the condition without IL-2) (Figure 5A and 5B). Cell viability remained between 85 - 99 % in cells cultured in the presence of IL-2 (Figure 5C), while among cells cultured without IL-2, only NK-92-SFFV-CAR.19-IL-15/IL-15Rα cells remained with viability between 80 - 95 % during the 21 days of culture (Figure 5D).

Thus, cells that were transduced with SEW-SFFV-CAR.19-IL-15/IL-15Rα vector could be expanded without IL-2 or with lower amounts of this interleukin, reducing the costs of culture.

### - CAR composition influences cytotoxic activity against CD19+ B cells

A broad-spectrum cytotoxicity and cell viability assays are currently used to determine the potency of T/NK-CAR cells. An ideal assay for the determination of viability and / or *in vitro* cytotoxicity should be rapid, safe, reliable, efficient, and cost-effective. Therefore, three different methods were tested: i) quantification by flow cytometry, ii) *Delphia Europium* assay and iii) *Incucyte*^{®} *Cytotoxicity Assay.*

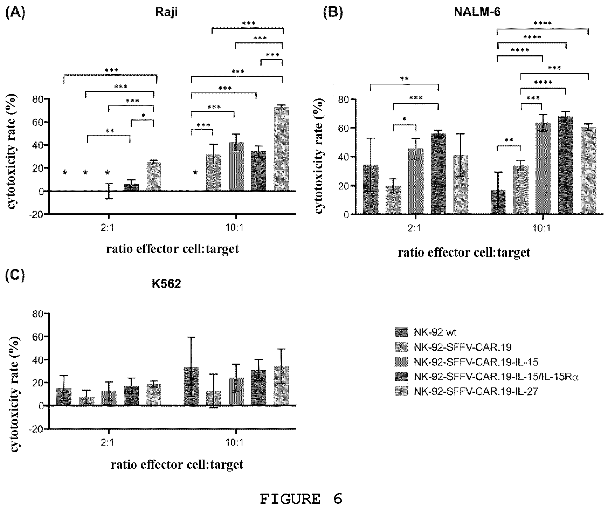

Figures 6A-C graphically represent the cytotoxic effect of NK-92-CAR cells against CD19+ (NALM-6 and Raji) and CD19- (K562) cells, wherein NK-92 wt, NK-92-SFFV-CAR.19, NK-92-SFFV-CAR.19-IL-15, NK-92-SFFV-CAR.19-IL-15/IL-15Rα and NK-92-SFFV-CAR.19- IL-27 were co-cultured with CD19+ tumor lines, which (A) refer to Raji (n = 3); (B) refer to NALM-6 (n = 6) and CD19- tumor line; and (C) refer to K562 (n = 6), at effector:target cell ratios of 2:1 and 10:1, for 5 hours. For these results, the One-way ANOVA statistical test was performed, with Tukey's multiple comparison post-test, and p values were considered: p < 0.05 (*), p < 0,01 (**); p < 0,0003 (***) and p < 0,0001 (****).

As shown in Figures 6A-C, in order to evaluate cytotoxicity by flow cytometry, NK-CAR.19 cells were incubated with the human cell lines CD19+ Raji (from LB) and NALM-6 (from ALL) and with the CD19-K562 tumor cell line (specificity control), at the ratio of effector:target cells of 2:1 and 10:1, for a period of 5 hours.

In co-cultivation with Raji cell line, it was noted that in the 2:1 ratio, only NK-92-SFFV-CAR.19-IL-27 was more cytotoxic than NK-92 cells wt and the other CAR.19 constructs are more cytotoxic than the unmodified NK-92 cells, and in addition, NK-92-SFFV-CAR.19-IL-27 cells showed more efficient cytotoxic activity against Raji cells than the NK-92-SFFV-CAR.19, NK-92-SFFV-CAR.19-IL-15 and NK-92-SFFV-CAR.19-IL-15/IL-15Rα constructs (Figure 6A). For the NALM-6 cell line, it was observed that only NK-92-SFFV-CAR.19-IL-15/IL-15Rα was more cytotoxic than NK-92 wt cells in the 2:1 ratio. However, at the 10:1 ratio all CAR.19 constructs were more cytotoxic than unmodified NK-92 cells, and in addition, NK-92-SFFV-CAR.19-IL-15, NK-92-SFFV-CAR.19-IL-15/IL-15Rα and NK-92-SFFV-CAR.19-IL-27 cells showed a more efficient antitumor effect against NALM-6 cells than NK-92 cells transduced with the SEW-SFFV-CAR.19 construct. This may indicate that cytokines may play some relevant role in the cytotoxicity of modified NK-92 cells (Figure 6B).

### - Fourth generation CARs induce secretion of cytokines in NK cells

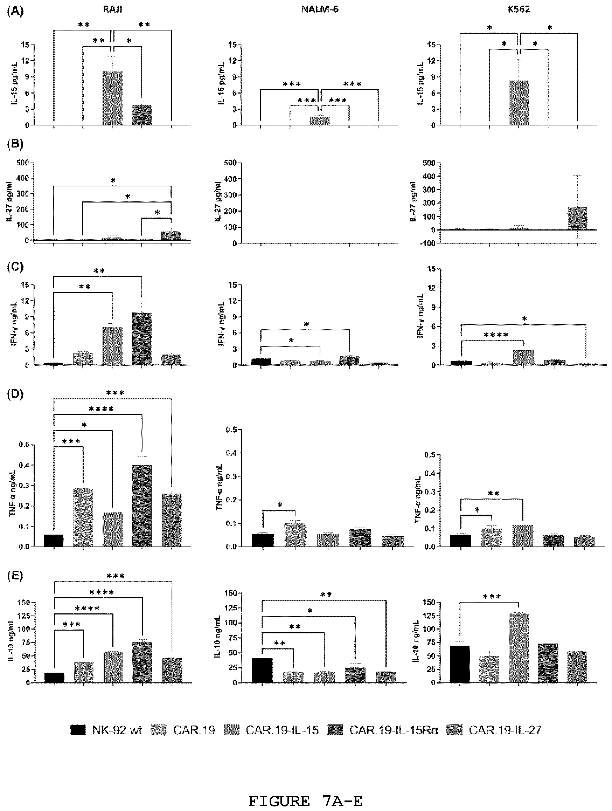

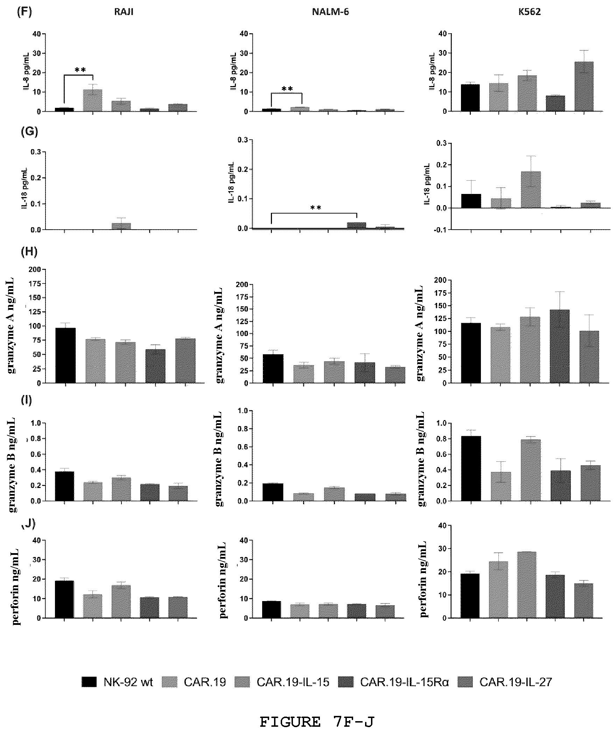

In order to evaluate the cytokines and other proteins involved in the cytotoxic action of NK-CAR cells, the proteins secreted after co-cultivation with the target cells (CD19+ and CD19-) were dosed using the Luminex MAGPIX system. In this assay, the cytokines IL-15, IL-27, IFN-γ, TNF-α, IL-10, IL-8, IL-18, granzyme A, granzyme B and perforin were evaluated (Figure 7).

It is worth noting that Figures 7A-J graphically represent the cytokines secreted in co-cultivation assay of effector cells NK-92 wt or NK-92 transduced with different constructs of CAR.19 and target cells Raji, NALM-6 and K562, wherein the legend of the mentioned graphs refers to effector cells as follows: to NK-92 wt (not transduced); CAR.19 refers to NK-92-SFFV-CAR.19; CAR.19-IL-15 refers to NK-92-SFFV-CAR.19-IL-15; CAR.19-IL-15Rα refers to NK-92-SFFV-CAR.19-L-15/IL-15Rα; and CAR.19-IL-27 refers to NK-92-SFFV-CAR.19-IL-27. For better understanding of the graphs in Figure 7, the following is reported to determine the concentration of (A) IL-15 (pg / ml), (B) IL-27 (pg / ml), (C) IFN-γ (ng / ml), (D) TNF-α (ng / ml), (E) IL-10 (ng / ml), (F) IL-8 (pg / ml) and (G) IL-18 (pg / ml) (H) Granzyme A (ng / ml) (I) Granzyme B (ng / ml) and (J) Perforin (ng / ml) in supernatant collected from effector cells co-cultured with Raji, NALM-6 and K562 target cells, respectively. For these results, One-way ANOVA statistical test with Tukey's multiple comparison post-test was performed and were considered the following p values: p < 0.05 (*), p < 0,01(**), p < 0,0003 (***) and p < 0,0001 (****).

Therefore, co-cultivation was performed with the effector cells NK-92 wt, NK-92-SFFV-CAR.19, NK-92-SFFV-CAR.19-IL-15, NK-92-SFFV-CAR.19-IL-15/IL-15Rα and NK-92-SFFV-CAR.19-IL-27 with the CD19 positive target cells Raji, NALM-6 and CD19 negative K562, in a 10:1 ratio, for a period of 5 hours.

### - Repeated stimulation differentially affects NK-CAR cell exhaustion

To evaluate the effects of repeated stimulation on NK-CAR cell variants, NK-92 wt cells, NK-92-SFFV-CAR.19, NK-92-SFFV-CAR.19-IL15, NK-92-SFFV-CAR.19-IL-15/IL-15Rα and NK-92-SFFV-CAR.19-IL-15Rα and NK-92-SFFV-CAR.19-IL-27 cells were co-cultured at a 1:2 effector:target cell ratio. IL-27 were co-cultured at an effector:target cell ratio of 1:2, with repeated exposures of the effector cells to the target cells (Raji CD19+) at times 0h, 24h and 48h. The evaluation of markers associated with cell exhaustion, LAG-3, PD-1, and Tim-3, was then determined by flow cytometry, and compared with NK-CAR cells maintained in the absence of target.

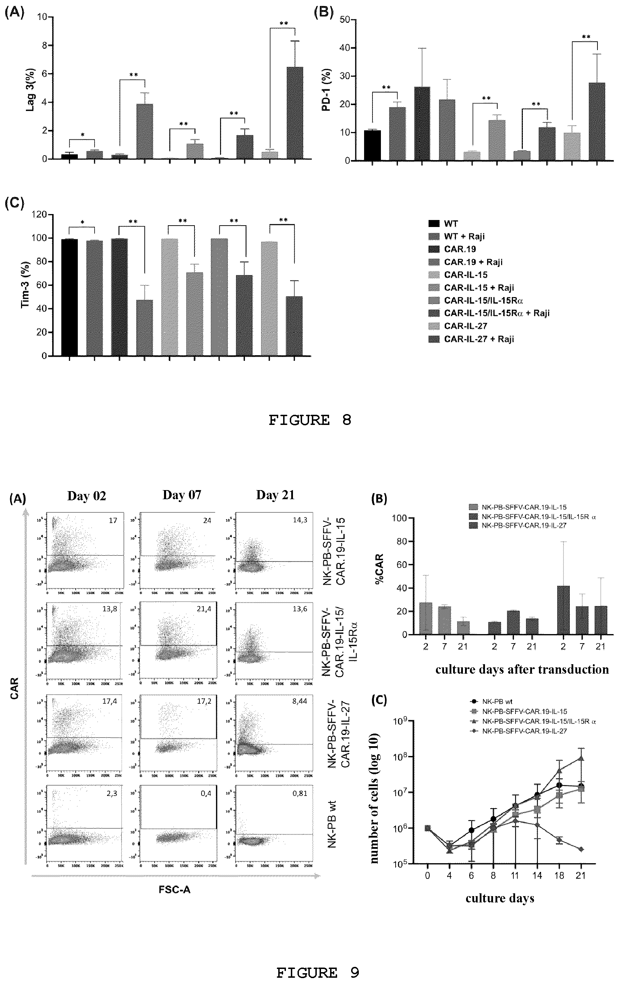

Figures 8A-C graphically represent the cell exhaustion (re-challenging) assay of the cells NK-92 wt, NK-92-SFFV-CAR.19, NK-92-SFFV-CAR.19-IL-15, NK-92-SFFV-CAR.19-IL-15/IL-15Rα, NK-92-SFFV-CAR.19-IL-27 exposed to the Raji target cell in a 1:2 ratio (effector cells: target cells), wherein the legend of the mentioned graphs refers to effector cells as follows: WT refers to NK-92 wt; CAR.19 refers to NK-92-SFFV-CAR.19; CAR-IL-15 refers to NK-92-SFFV-CAR.19-IL-15; CAR-IL-15/IL-15Rα refers to NK-92-SFFV-CAR.19-IL-15/IL-15Rα and IL-27 refers to NK-92-SFFV-CAR.19-IL-27, which were co-cultured in the presence or absence of Raji target cells. In addition, new target cells were added at a 1:2 ratio (effector:target) at 0h, 24h and 48h, and the labeling with the antibodies (A) LAG-3, (B) PD-1 and (C) Tim-3 was performed 72 hours after the beginning of the assay. For these results, Student's t statistical test was performed and were considered the significant p values < 0.05 (*) and p < 0.01(**).

Therefore, NK-92 wt, NK-92-SFFV-CAR.19, NK-92-SFFV-CAR.19-IL15, NK-92-SFFV-CAR.19-IL-15/IL-15Rα and NK-92-SFFV-CAR.19-IL-27 effector cells showed increased expression of LAG-3 when exposed to target cells for 72h. Among the target cells, NK-92-SFFV-CAR.19-IL-27 cells showed higher LAG-3 expression than the other effector cells (Figure 8A).

As for the cellular marker PD-1, except for NK-92-SFFV-CAR.19 cells, all the others showed higher PD-1 expression after repeated exposures to the target cells (Figure 8B). PD-1 expression was higher in NK-92-SFFV-CAR.19-IL-27 cells than in the other effector cells when exposed to Raji (Figure 8B).

For all NK-CAR cell variants, repeated stimulation led to decreased surface expression of Tim-3, which showed high expression even without stimulation with the target cells (Figure 8C).

As for the cellular marker PD-1, except for NK-92-SFFV-CAR.19 cells, all the others showed higher PD-1 expression after repeated exposures to the target cells (Figure 19B). PD-1 expression was higher in NK-92-SFFV-CAR.19-IL-27 cells than in the other effector cells when exposed to Raji (Figure 8B).

For all NK-CAR cell variants, repeated stimulation led to decreased surface expression of Tim-3, which showed high expression even without stimulation with the target cells (Figure 8C).

### - Generation of peripheral blood NK-CAR cells and evaluation of their antitumor capacity

In parallel to the NK-92-CAR cell generations, the effect of the constructs of the present invention on primary NK cells isolated from PB.

PB NK cells were isolated by CD56-negative immunomagnetic selection. Next, NK cells were activated with beads and transduced with SEW-SFFV-CAR.19-IL-15, SEW-SFFV-CAR.19-IL-15/IL-15Rα and SEW-SFFV-CAR.19-IL-27 (MOI 200) and cultured with NK MACS (Miltenyi) medium supplemented with 5% human AB serum, IL-2 (1000 IU / mL) and IL-21 (20 ng / ml).

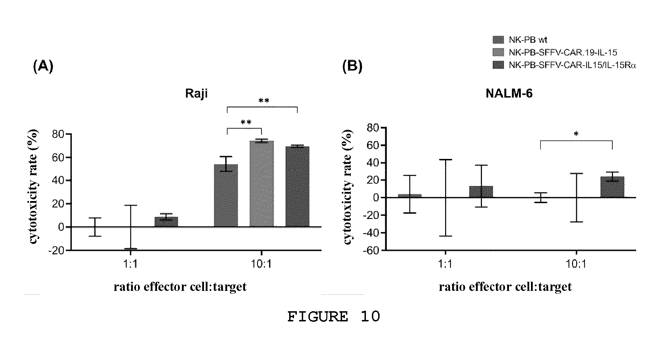

Figures 9A-C graphically represent the transduction of primary PB NK cells with SEW-SFFV-CAR.19-IL-15, SEW-SFFV-CAR.19-IL-15/IL-15Rα and SEW-SFFV-CAR.19-IL-27 vectors and cell proliferation. IL-27 and cell proliferation, wherein (A) is shown an analysis of CAR.19 expression by flow cytometry of NK-PB-SFFV-CAR.19-IL-15, NK-PB-SFFV-CAR.19-IL-15/IL-15Rα, NK-PB-SFFV-CAR.19-IL-15, NK-PB-SFFV-CAR.19-IL-15/IL-15Rα and NK-PB-SFFV-CAR.19-IL-15/IL-15Rα cells. IL-27 and NK-PB wt (unmodified NK cells control for nonspecific binding) from peripheral blood on days 02, 07 and 21; (B) is shown the percentage of CAR.19-positive cells for NK-PB-SFFV-CAR.19- IL-15 (2 donors), NK-PB-SFFV-CAR.19-IL-15/IL-15Rα (1 donor, duplicate) and NK-PB-SFFV-CAR.19-IL-27 (2 donors); and in (C) is shown the cell proliferation of NK-PB wt and transduced with CAR.19 vectors co-expressing IL-15, IL-15/IL-15Rα or IL-27.

Therefore, the transduced cells showed a decrease in CAR expression over the 21-day period (Figure 9A and 9B), except the NK-PB-SFFV-CAR.19-IL-15/IL-15Rα cells, which showed a slightly more constant expression. Regarding cell proliferation, cells transduced and not transduced with CAR.19 vector, with the exception of NK-PB-SFFV-CAR.19-IL-27 cells, showed a similar cell proliferation with the unmodified NK cells (Figure 9C). The NK-PB-SFFV-CAR.19-IL-27 cells stopped growing from day 11 and from then on, the total number of cells started to decrease (Figure 9C).

Next, the cytotoxic capacity of NK-PB-SFFV-CAR.19-IL-15 and NK-PB-SFFV-CAR.19-IL-15/IL-15Rα cells was evaluated. The NK-PB-SFFV-CAR.19-IL-27 cells were not evaluated because they did not have sufficient cell numbers due to the decay in cell growth (Figure 9C).

Figures 10A-B graphically represent the cytotoxicity assay for NK-PB-SFFV-CAR.19-IL-15, NK-PB-SFFV-CAR.19-IL-15/IL-15Rα and NK-PB wt cells against CD19 positive tumor cells (triplicate), wherein the cytotoxic effect of PB NK cells transduced with CAR.19 containing IL-15 or IL-15 with alpha receptor against tumor target cells was evaluated by cytotoxicity assay by flow cytometry method. Thus, NK-PB wt, NK-PB-SFFV-CAR.19-IL-15 and NK-PB-SFFV-CAR.19-IL-15/IL-15Rα cells were co-cultured with CD19+ tumor cell lines (A) Raji and (B) NALM-6 at effector:target cell ratios of 1:1 and 10:1, for 2 hours. For (A) and (B), One-way ANOVA statistical test was used, with Tukey's multiple comparison post-test. P values < 0.05 (*) and p < 0.005 (**).

Thus, as shown in Figures 10A-B, to assess cytotoxicity, NK-CAR.19 cells (7 days after transduction) were incubated with the human CD19+ cell lines Raji and NALM-6, at effector:target cell ratios of 1:1 and 10:1, for a period of 2 hours (Figure 10A and 10B). CAR expression in these cells at the time of the co-cultivation experiment was 23.6% for NK-PB-SFFV-CAR.19-IL-15 and 21% for NK-CB-SFFV-CAR.19-IL-15/IL-15Rα. For Raji strain, both NK-PB-SFFV-CAR.19-IL-15 and NK-PB-SFFV-CAR.19-IL-15/IL-15Rα are more cytotoxic than NK-PB wt cells at 10:1 ratio (Figure 10A). For NALM-6 strain, NK-PB-SFFV-CAR.19-IL-15/IL-15Rα cells were more cytotoxic than non-transduced cells (Figure 10B). NK-PB-SFFV-CAR.19-IL-15 cells showed no cytotoxicity against NALM-6.

Therefore, it was concluded that PB NK cells have a higher transduction efficiency (Figure 9B) than NK-92 cells (Figures 3A-D). NK-PB-SFFV-CAR.19-IL15 and IL-15/IL-15Rα cells are cytotoxic to Raji cells and only NK-PB-SFFV-CAR.19-IL15/IL-15Rα cells are cytotoxic to NALM-6 cells.

### - Antitumor potential of NK-CAR cells in vivo

To evaluate the *in vivo* antineoplastic potential of NK-CAR cells, 15 immunodeficient NOD-scid gamma lineage (NSG) mice received 4 x 10⁴ RAJI-luc cells intravenously (lateral tail vein). After 4 days of tumor cell injection, animals were randomly assigned into 6 groups that received 7 x 10⁶ cells intravenously (A to E): A) NK-92 wt (n = 3), B) NK-92-SFFV-CAR.19 (n = 2), C) NK-92-SFFV-CAR.19-IL-15 (n = 2), D) NK-92-SFFV-CAR.19-IL-15/IL-15Rα (n = 3) and E) NK-92-SFFV-CAR.19-IL-27 (n = 3). The control group (F) (n = 2) received only PBS 1x.

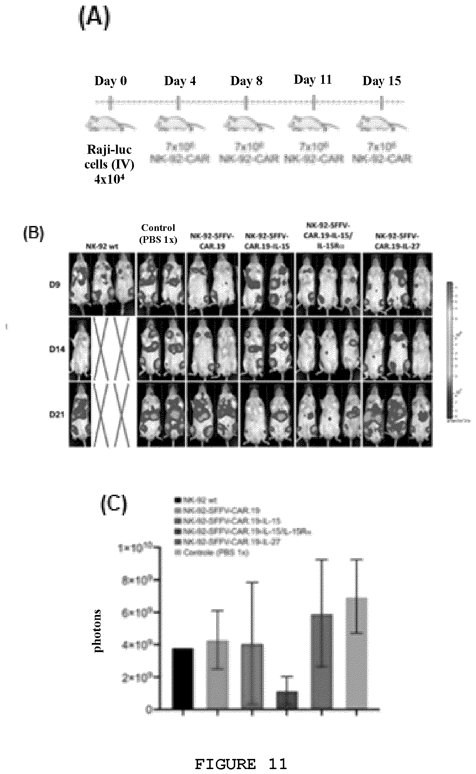

Figures 11A, 11C and 11E show the evaluation of the cytotoxic potential of NK-92-SFFV-CAR.19 (4 constructs) and NK-92 wt in NSG mouse models with infusion of 4x104 Raji-luc tumor cells (systemic lymphoma model), wherein a Figure 11A refers too scheme of the animal experiment indicating the day of tumor cell infusion and treatment doses, a Figure 11C refers to evaluation of tumor burden (photons/s) on day 21 after infusion of Raji-Luc cells, and Figure 11E are images of the evaluation of bioluminescence intensity (photons / second) on day 9, 14 and 21 in the treated (NK-92 wt, NK-92-SFFV-CAR-19, NK-92-SFFV-CAR-19-IL-15, NK-92-SFFV-CAR-19-IL-15/IL-15Rα and NK-92-SFFV-CAR-19-IL-27) and untreated (Control - PBS 1X) groups. in IVIS *Lumina System* equipment (Perkin Elmer).

Therefore, the animals received 4 infusions (doses) of 7 x 10⁶ NK cells mentioned above, on days 4, 8, 11 and 15 after tumor induction (Figure 11A). During and after treatment, the tumor burden was monitored by quantification of the bioluminescence (IVIS Lumina System, Perkin Elmer) .

Bioluminescent signal analyses demonstrated that there was a difference in tumor burden between the groups treated with NK-92-CAR cells and the control (PBS 1X) (Figure 11B and 11C). Among the treatments, the group treated with NK-92-SFFV-CAR.19-IL-15/IL-15Rα cells showed the lowest rate of tumor progression on day 21 (Figure 11C) and was the group that had the last animal to die (day 28).

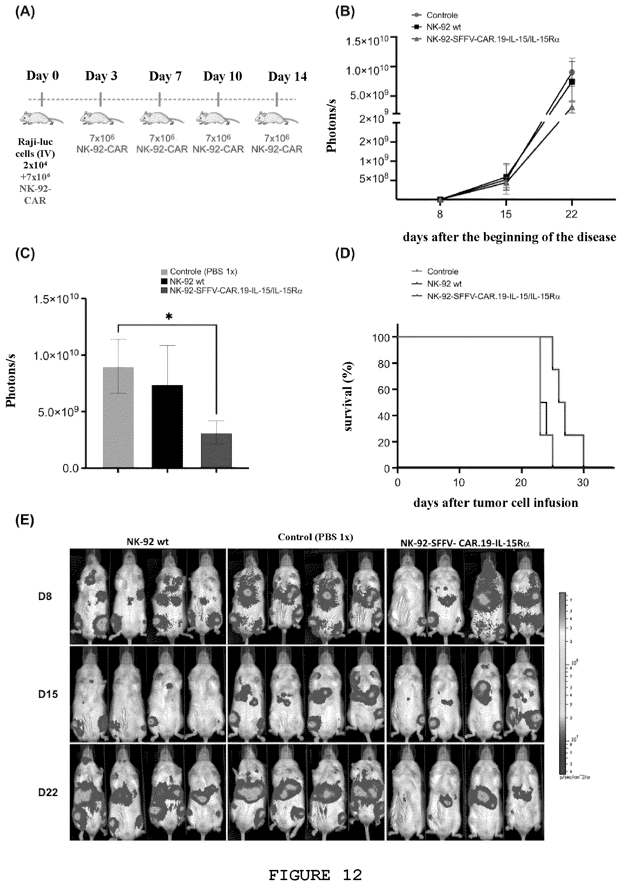

To try to observe some effect of NK-92-CAR cells *in vivo* and to evaluate the cytotoxicity of these cells, the tumor burden was decreased and the number of infusions of NK-92-CAR cells was increased (Figure 12A).

Figures 12A-E show the evaluation of the cytotoxic potential of NK-92-SFFV-CAR.19-IL-15 / IL-15Rα and NK-92 wt in NSG mouse models after IV infusion of 2 x 10⁴ Raji-luc tumor cells (systemic lymphoma model), wherein (A) refers to the schematic of animal experiment indicating the day of tumor infusion and the days of treatment doses; (B) graphically represents of the bioluminescence intensity (photons/s) on days 8, 15 and 22 of the treated (NK-92 wt or NK-92-SFFV-CAR-19-IL-15/IL-15Rα) and untreated (Control - PBS 1X) groups, wherein One-way ANOVA statistical test (p>0.05) was used; (C) graphically represents of Bioluminescence intensity (photons/s) from day 22 only of the treated (NK-92 wt and NK-92-SFFV-CAR-19-IL-15/IL-15Rα) and untreated (Control - PBS 1X) groups, wherein One-way ANOVA statistical test and Tukey's multiple comparison post-test (p < 0.05) were used; (D) graphically refers to the survival curve of animals after treatment with genetically modified and unmodified NK-92 cells, wherein the log-rank test (p < 0.05) was performed; and (E) are images of the evaluation of bioluminescence intensity of treated (NK-92 wt and NK-92-SFFV-CAR-19-IL-15/IL-15Rα) and untreated animals (Control - PBS 1X) on days 8, 15 and 22 obtained by IVIS Lumina System equipment (Perkin Elmer).

Accordingly, a new experiment was performed with an initial number of 2 x 10⁴ Raji-Luc cells, infused via the tail vein. Since NK-92-SFFV-CAR.19-IL-15/IL-15Rα cells were apparently shown to be better than NK-92 cells modified with the other CAR.19 constructs, these cells were chosen to evaluate the efficacy of genetically modified NK-92 cells. In addition, 5 doses were applied instead of 4 and the first dose was infused on day 0. The control group received PBS 1x (n = 4), the groups treated with NK-92 wt (n = 4) and treated with NK-92-SFFV-CAR.19-IL-15/IL-15Rα (n = 4) received five infusions of 7 x 10⁶ NK-CAR cells on days 0, 3, 7, 10 and 14 after tumor induction. The results showed a small difference in the survival curve of NK-92-CAR.19-IL-15/IL-15Rα treated animals compared to animals in the NK-92 wt and control (PBS 1x) group (log-rank test, p=0.02) (Figure 12D). Bioluminescence analysis showed no differences between treated animals and controls (Figure 12B). However, when analyzing day 21 after tumor infusion alone, the group treated with NK-92-SFFV-CAR.19-IL-15/IL-15Rα cells had a lower tumor burden than the control group (PBS 1x) but was not lower than the group treated with NK-92 wt (Figure 12C).

Therefore, it is concluded that NK-92-CAR.19 cells, even though they have *in vitro* antitumor potential, were not as effective *in vivo.* This can be explained by the difficulty of migration of NK-CAR cells to the areas most affected by the tumor, such as liver, spleen, and bone marrow. However, again, the tests presented here showed a slight decrease in tumor progression in animals treated with NK-92-SFFV-CAR-19-IL-15/IL-15Rα.

In view of the above, the constructs described herein reveal that the antitumor activity is enhanced by CAR-NK cells co-expressing interleukin-15 fused to its receptor RA (IL-15 / IL15 RA) and interleukin-27 (IL-27) and that the persistence and proliferation of CAR-NK cells are especially enhanced.

Furthermore, the results described herein are promising that the gene transfer modality and expression of the interleukins IL-15RA and IL-27 together with CAR on NK cells can be extended to a gene transfer modality and their expression on immune cells other than NK cells, such as T cells, monocytes, macrophages, and dendritic cells.

The invention further refers to the following aspects, as defined in the following numbered items:
1. Nucleic acid sequence encoding a chimeric antigen natural killer cell receptor (NK-CAR), wherein the NK-CAR comprises:
   (a) an anti-CD19 single-chain variable fragment (scFv);
   (b) a CD8 transmembrane domain;
   (c) a 4-1BB co-stimulatory domain; and
   (d) an intracellular T-cell signaling domain of CD3ζ,
   wherein the said nucleic acid sequence further comprises an autocleavage peptide and a transgene encoding at least one selected from the group consisting of interleukin-15 with its receptor RA (IL-15RA) and interleukin-27 (IL-27).
2. Nucleic acid sequence, according to item 1, wherein the scFV anti-CD19 comprises the amino acid sequence as set out in the SEQ ID NO: 1, wherein it is encoded by the nucleotide sequence as set out in SEQ ID NO: 2.
3. Nucleic acid sequence, according to item 1, wherein the transmembrane domain comprises the amino acid sequence as set out in the SEQ ID NO: 3, wherein it is encoded by the nucleotide sequence as set out in SEQ ID NO: 4.
4. Nucleic acid sequence, according to item 1, wherein the co-stimulatory domain comprises the amino acid sequence as set out in the SEQ ID NO: 5, wherein it is encoded by the nucleotide sequence as set out in SEQ ID NO: 6.
5. Nucleic acid sequence, according to item 1, wherein the intracellular T-cell signaling domain of CD3ζ comprises the amino acid sequence as set out in the SEQ ID NO: 7, wherein it is encoded by the nucleotide sequence as set out in SEQ ID NO: 8.
6. Nucleic acid sequence, according to item 1, wherein the heterologous autocleavage peptide is preferably the T2A comprising the amino acid sequence as set out in SEQ ID NO: 9, wherein it is encoded by the nucleotide sequence as set out in SEQ ID NO: 10.
7. Nucleic acid sequence, according to item 1, wherein the transgene encoding at least one selected from the group consisting of interleukin-15 with its receptor RA (IL-15RA) and interleukin-27 (IL-27) comprises the nucleotide sequence as set out in SEQ ID Nos: 11 and 12, respectively, wherein the IL-15 is linked to its receptor IL-15Rα by a linker comprising the nucleotide sequence as set out in SEQ ID NO: 20; and the EBI3 subunit of the IL-27 is linked to the subunit IL-27p28 of the IL-27 by a elastin linker comprising the nucleotide sequence as set out in SEQ ID NO: 19.
8. Nucleic acid sequence, according to item 1, wherein the IL-15RA and IL-27 encoded by such transgene comprises the amino acid sequences as set out in SEQ ID Nos: 13 and 14, respectively.
9. Nucleic acid sequence, according to any of the items 1 to 8, wherein the NK-CAR is a fourth generation CAR designed to secrete a cytokine along with CAR signaling in the target tumor tissue.
10. Nucleic acid sequence, according to any of the items 1 to 8, wherein it comprises the NK-CAR nucleotide sequences selected from the group consisting of SEQ ID NO: 15 referring to CAR Cd19/IL15RA and SEQ ID NO: 16 referring to CAR Cd19/IL27.
11. chimeric antigen natural killer cell receptor (NK-CAR) polypeptide wherein it comprises:
   (a) an anti-CD19 single-chain variable fragment (scFv);
   (b) a CD8 transmembrane domain;
   (c) a 4-1BB co-stimulatory domain; and
   (d) an intracellular T-cell signaling domain of CD3ζ,
   wherein such polypeptide further comprises an autocleavage peptide and a at least one cytokine selected from the group consisting of interleukin-15 with its receptor RA (IL-15RA) and interleukin-27 (IL-27).
12. NK-CAR polypeptide, according to item 11, wherein it comprises the amino acid sequences selected from the group consisting of SEQ ID NO: 17 referring to CAR Cd19/IL15RA and SEQ ID NO: 18 referring to CAR Cd19/IL27.
13. Vector wherein it comprises the nucleic acid sequence as defined in any of the items 1 to 10.
14. Vector, according to item 13, wherein the nucleic acid sequence is as set out in SEQ ID NO: 14 or SEQ ID NO: 15.
15. Vector, according to item 13, wherein it is a lentiviral vector.
16. *In vitro* method of obtaining a cell wherein it comprises the following steps:
   a) transforming a cell with the vector as defined in any of the claims 13 to 15; and
   b) culturing such transformed cells under conditions of cell growth.
17. *In vitro* method, according to item 16, wherein such cell is a Natural-Killer (NK) cell.
18. Use of the nucleic acid sequence as defined in any of the items 1 to 10, of the NK-CAR polypeptide as defined in item 11 or 12, or of the vector as defined in any of the items 13 to 15 wherein it is for the preparation of a drug to treat the cancer.
19. Use, according to item 18, wherein the cancer is selected from B-cell cancers, such as lymphoma and leukemia.
20. Use, according to item 18, wherein it is for treating cancer in an allogeneic therapy.
21. Pharmaceutical composition wherein it comprises:
   (i) nucleic acid sequence as defined in any of the claims 1 to 10, or
   (ii) NK-CAR polypeptide as defined in claim 11 or 12, or
   (iii) vector as defined in any of the claims 13 to 15, and
   (iv) a pharmaceutically acceptable vehicle.

Thus, the embodiments presented in the present invention do not limit the totality of possibilities, and it will be understood that several omissions, substitutions, and changes can be made by a person skilled in the art, without departing from the spirit and scope of the present invention.

It should also be understood that the drawings are not necessarily to scale, but that they are only conceptual in nature. The intent is therefore to be limited, as indicated by the scope of the attached claims.

It is expressly provided that all combinations of elements which perform the same function in substantially the same way to achieve the same results are within the scope of the invention. Substitutions of elements from one described embodiment to another are also fully intended and contemplated.

A person skilled in the art will value the knowledge presented herein and will be able to reproduce the invention in the presented embodiments and in other variants, falling within the scope of the claims.

## Claims

1. Nucleic acid sequence **characterized in that** it encodes a chimeric antigen natural killer cell receptor (NK-CAR), wherein the NK-CAR comprises:
(a) an anti-CD19 single-chain variable fragment (scFv);
(b) a CD8 transmembrane domain;
(c) a 4-1BB co-stimulatory domain; and
(d) an intracellular T-cell signaling domain of CD3ζ,
wherein the said nucleic acid sequence further comprises an autocleavage peptide and a transgene encoding at least one selected from the group consisting of interleukin-15 with its receptor RA (IL-15RA) and interleukin-27 (IL-27).

2. Nucleic acid sequence, according to claim 1, **characterized in that** the scFV anti-CD19 comprises the amino acid sequence as set out in the SEQ ID NO: 1, wherein it is encoded by the nucleotide sequence as set out in SEQ ID NO: 2.

3. Nucleic acid sequence, according to claim 1, **characterized in that** the transmembrane domain comprises the amino acid sequence as set out in the SEQ ID NO: 3, wherein it is encoded by the nucleotide sequence as set out in SEQ ID NO: 4.

4. Nucleic acid sequence, according to claim 1, **characterized in that** the co-stimulatory domain comprises the amino acid sequence as set out in the SEQ ID NO: 5, wherein it is encoded by the nucleotide sequence as set out in SEQ ID NO: 6.

5. Nucleic acid sequence, according to claim 1, **characterized in that** the intracellular T-cell signaling domain of CD3ζ comprises the amino acid sequence as set out in the SEQ ID NO: 7, wherein it is encoded by the nucleotide sequence as set out in SEQ ID NO: 8.

6. Nucleic acid sequence, according to claim 1, **characterized in that** the heterologous autocleavage peptide is preferably the T2A comprising the amino acid sequence as set out in SEQ ID NO: 9, wherein it is encoded by the nucleotide sequence as set out in SEQ ID NO: 10.

7. Nucleic acid sequence, according to claim 1, **characterized in that** the transgene encoding at least one selected from the group consisting of interleukin-15 with its receptor RA (IL-15RA) and interleukin-27 (IL-27) comprises the nucleotide sequence as set out in SEQ ID Nos: 11 and 12, respectively, wherein the IL-15 is linked to its IL-15Rα receptor by a linker comprising the nucleotide sequence as set out in SEQ ID NO: 20; and the EBI3 subunit of the IL-27 is linked to the subunit IL-27p28 of IL-27 by a elastin linker comprising the nucleotide sequence as set out in SEQ ID NO: 19.

8. Nucleic acid sequence, according to claim 1 or 7, **characterized in that** the IL-15RA and IL-27 encoded by the said transgene comprises the amino acid sequences as set out in SEQ ID Nos: 13 and 14, respectively.

9. Nucleic acid sequence, according to any of the claims 1 to 8, **characterized in that** the NK-CAR is a fourth generation CAR designed to secrete a cytokine together with CAR signaling in the target tumor tissue.

10. Nucleic acid sequence, according to any of the claims 1 to 8, **characterized in that** it comprises the NK-CAR nucleotide sequences selected from the group consisting of SEQ ID NO: 15 referring to CAR Cd19/IL15RA and SEQ ID NO: 16 referring to CAR Cd19/IL27.

11. Polypeptide of the chimeric antigen natural killer cell receptor (NK-CAR) **characterized in that** it comprises:
(a) an anti-CD19 single-chain variable fragment (scFv);
(b) a CD8 transmembrane domain;
(c) a 4-1BB co-stimulatory domain; and
(d) an intracellular T-cell signaling domain of CD3ζ,
wherein such polypeptide further comprises an autocleavage peptide and a at least one cytokine selected from the group consisting of interleukin-15 with its receptor RA (IL-15RA) and interleukin-27 (IL-27).

12. NK-CAR polypeptide, according to claim 11, **characterized in that** it comprises the amino acid sequences selected from the group consisting of SEQ ID NO: 17 referring to CAR Cd19/IL15RA and SEQ ID NO: 18 referring to CAR Cd19/IL27.

13. Vector **characterized in that** it comprises the nucleic acid sequence as defined in any of the claims 1 to 10.

14. Vector, according to claim 13, **characterized in that** the nucleic acid sequence is as set out in SEQ ID NO: 14 or SEQ ID NO: 15.

15. Vector, according to claim 13, **characterized in that** it is a lentiviral vector.

16. *In vitro* method of obtaining a cell **characterized in that** it comprises the following steps:
a) transforming a cell with the vector as defined in any of the claims 13 to 15; and
b) culturing such transformed cells under conditions of cell growth.

17. *In vitro* method, according to claim 16, **characterized in that** such cell is a Natural-Killer (NK) cell.

18. Use of the nucleic acid sequence as defined in any of the claims 1 to 10, of the NK-CAR polypeptide as defined in claim 11 or 12, or of the vector as defined in any of the claims 13 to 15 **characterized in that** it is for the preparation of a drug to treat the cancer.

19. Use, according to claim 18, **characterized in that** the cancer is selected from B-cell cancers, such as lymphoma and leukemia.

20. Use, according to claim 18, **characterized in that** it is to treat the cancer in an allogeneic therapy.

21. Pharmaceutical composition **characterized in that** it comprises:
(i) nucleic acid sequence as defined in any of the claims 1 to 10, or
(ii) NK-CAR polypeptide as defined in claim 11 or 12, or
(iii) vector as defined in any of the claims 13 to 15, and
(iv) a pharmaceutically acceptable vehicle.
